# EUROPEAN PATENT APPLICATION

(11) **EP 4 467 073 A1**
(43) Date of publication of application: **27.11.2024**
(21) Application number: 24168857.1
(22) Date of filing: 05.04.2024
(51) Int. Cl.: A61B 5/107, A61B 90/00

(54) **PELVIC TILT VERIFICATION DEVICE**

(30) Priority: 05.04.2023 NL 2034507
(71) Applicant: Massmedica S.A., 02-972 Warschau (PL); Orpidem B.V., 6671 DC Zetten (NL)
(72) Inventor: VERMAAT, Jan, 6671 DC Zetten (NL)
(74) Representative: Verhees, Godefridus Josephus Maria

(57) **Abstract**

The pelvic tilt verification device 1 has a circular bubble spirit level 3 mounted on a spirit level carrier 5. The verification device 1 further has three pelvic contact elements 7 formed by straight bars provided with markings. These contact elements 7 are slidable in through holes arranged in slides 9. These slides are attached to guide elements 11 which are also formed by straight bars provided with markings. These guide elements are slidable in through holes arranged in the spirit level carrier 5. Thus, the slides 9 are slidable in a first direction with respect to the spirit level carrier 5 and in a second direction with respect to the pelvis contact elements 7. The first and second directions are at right angles to each other. The slides 9 are provided with fixing means 13 to fix them to the pelvis contact elements 7.

## Description

### Technical field of the invention

The invention relates to a pelvic tilt verification device for determining a possible difference between the original functional tilt of a patient's pelvis and the tilt when positioned on an operating table to undergo hip surgery. Proper acetabular component positioning is one of the mandatory aspects for a successful functional outcome in total hip surgery. Therefore, prior to any acetabular component preplanning the patient specific functional pelvic tilt angle should be recorded and compared with the actual tilt the moment the patient is positioned on the operating table to undergo surgery. In case a difference between the original functional tilt and the actual tilt and rotation is noticed the pelvis can be adjusted accordingly if felt necessary. Alternatively it may be decided to adjust the pre-planned cup inclination and/or version angle according to the pelvic tilt and rotation findings.

### Background of the invention

When applying an acetabular cup into a patient's surgically-prepared acetabulum, it is essential that the orientation of the pelvis is equal to the orientation on which basis the version and inclination planning of the acetabular component was performed as any difference may strongly influence the functional clinical outcome. Functional pelvic tilt may differ considerably from the actual tilt when a patient is positioned on the operating table to undergo surgery. In the supine position, the pelvic tilt is likely to change along the transverse axis whereas in the lateral position the pelvic tilt is likely to change along the vertical axis. The pelvis may then also demonstrate internal or external rotation along the longitudinal axis.

A pelvic tilt orientation verification device according to the preamble of claim 1 is known from JP 2004 89653 A. In this known device the frame is fixed to the bed on which the patient is lying when he/she undergoes hip surgery. The pelvis contact elements can be adjusted with respect to two guide elements that are slidably connected to the fixed frame.

### Summary of the invention

An object of the present invention is to provide a pelvic tilt verification device with which the tilt of the pelvis can be recorded more easily. To this end the pelvic tilt verification device according to the invention is characterized in that:
- one of the guide elements is at an angle with respect to the other guide element(s),
- the frame is a spirit level carrier,
- the spirit level is a bubble spirit level which is mounted on the spirit level carrier, and
- the slides are slidable along the pelvic contact elements.

Preferably, one of the guide elements is at right angle to the other guide element or guide elements (in case the device has more than two guide elements). With this pelvic tilt verification device the functional tilt of the pelvis of a patient can be recorded during a clinical examination, when taking x-rays (or other imaging) being positioned in an applicable surgical position and compared with the actual tilt when positioned on an operating table to undergo surgery. So, with the device according to the invention a quick and reliable verification of a possible change in the pelvic tilt can be obtained, minimising the guesswork for acetabular cup placement.

It may not always be possible to fully adjust the tilt of the pelvis, in which case the verification device may inform the surgeon about the possible need for adjustment of the pre-planned acetabular version and/or inclination angle.

Before starting the actual surgery the tilt verification device has to be applied to the patient using the settings as recorded during the original functional tilt measurement. In case when using these original device settings the air bubble of the indicator is out of center, the pelvic tilt of the patient can be adjusted or the slides of the device can be adjusted such that the air bubble is in the center. The adjustment can then be translated to a deviation of the original pelvic tilt angle allowing for adjusting the pre-operatively planned acetabular cup version and/or inclination angle.

Now the pelvic tilt verification device should be removed and the surgical hip joint procedure can be performed in the routine manner.

Preferably, the slides are attached to guide elements which are slidable in the spirit level carrier or the slides are slidable along guide elements mounted on the spirit level carrier. The first and second directions are, preferably, at right angles to each other.

Preferably, the device comprises three guide elements which are slidable in the spirit level carrier and the slides are attached to guide elements.

In an embodiment of the pelvic tilt verification device the pelvic tilt verification device provides fixation for securing the slides and/or the guide elements with respect to the pelvic contact elements and/or the spirit level carrier and the pelvic contact elements. All guiding and elongated elements are provided with markings.

The slides can be locked and secured in any given position and the pelvic contact elements are provided with markings to allow for calibration and or registration of pelvic tilt angles.

Preferably, the pelvic contact elements and the guiding elements are formed by straight bars, wherein two of the guiding elements are parallel and the center line between these two parallel bars correspondents with the center of the spirit level, and the other guiding element is perpendicular to said two parallel guiding elements and is in line with the center of the spirit level. Preferably all bars are designed to prevent rotation along their longitudinal axis and to allow for easy securing in any given position by means of a locking mechanism.

The pelvic tilt verification device can be applied to a patient operated in the lateral or supine position.

### Brief description of the drawings

The invention will be further elucidated below on the basis of drawings. These drawings show embodiments of the pelvic tilt verification device according to the present invention. In the drawings:
Figure 1 shows the pelvic tilt verification device arranged on a pelvis in perspective view;
Figure 2 shows the pelvic tilt verification device separately; and
Figure 3 shows the pelvic tilt verification device arranged on a pelvis in top view.

### Detailed description of the drawings

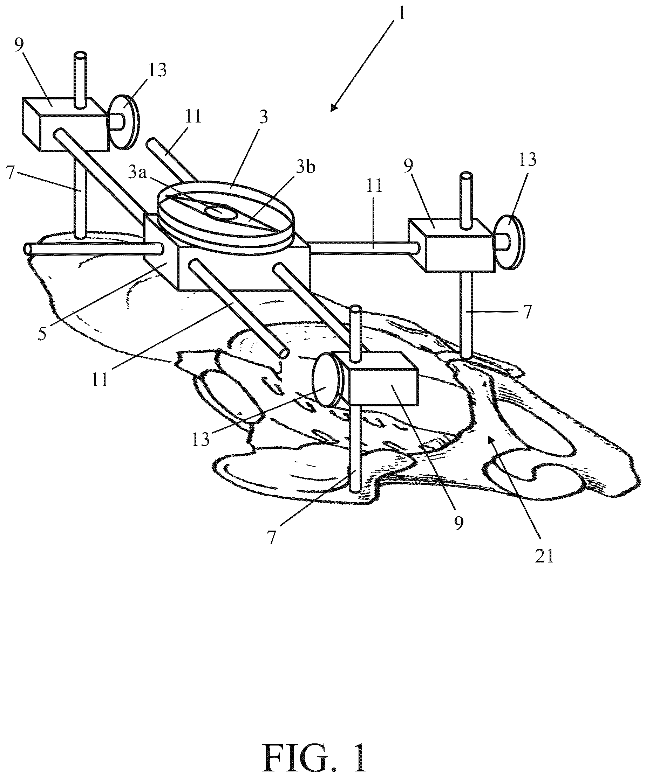

In figure 1 an embodiment of the pelvic tilt verification device according to the invention is shown in perspective view in a position arranged on a pelvis. The pelvic tilt verification device 1 has a circular bubble spirit level 3 mounted on a spirit level carrier 5. The verification device 1 further has three pelvis contact elements 7 formed by straight bars provided with markings. These contact elements 7 are slidable in through holes arranged in slides 9. These slides 9 are attached to guide elements 11 which are also formed by straight bars provided with markings. These guide elements 11 are slidable in through holes arranged in the spirit level carrier 5. Thus, the slides 9 are slidable in a first direction with respect to the spirit level carrier 5 and in a second direction with respect to the pelvic contact elements 7. The first and second directions are at right angles to each other.

The slides 9 are provided with fixing means 13 to fix them to the pelvic contact elements 7 and/or the spirit level carrier. Also the spirit level carrier can be provided with a locking mechanism (not shown in the drawings) to fix the slides to the pelvic contact elements.

If the skew of the device is too great (for example, in case a patient is lying on his/her side), the spirit level cannot indicate the degree of skew. The spirit level can indicate a maximum of approximately 5 degrees of skew of the spirit level. The air bubble of the spirit level is stopped by the side wall of the fluid chamber and no determination of the position is possible. In order to be able to determine the orientation of the pelvis even when the pelvis is inclined at a greater angle and therefore also of the device, the carrier 5 is provided with a mechanism (not shown in the drawings) to level the spirit level at an angle to the carrier so that the device can also be used in that case.

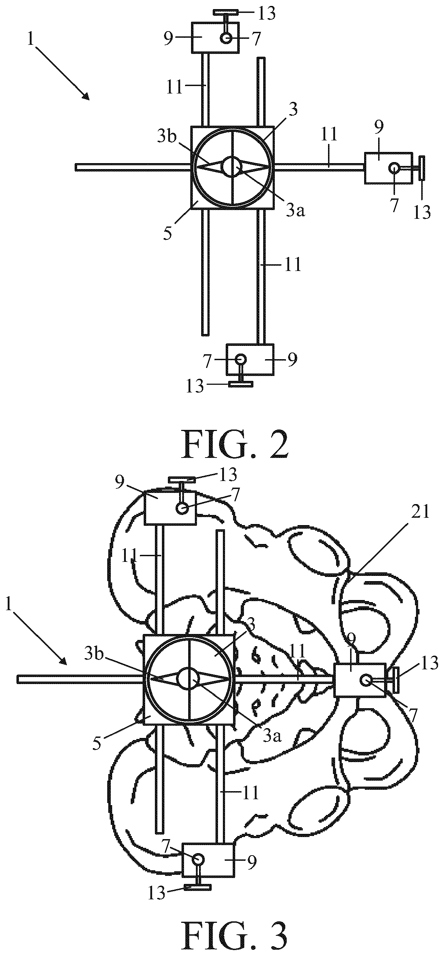

In figure 2 the pelvic tilt verification device is shown in top view and figure 3 shows the pelvic tilt verification device 1 being arranged on a pelvis 21 of which the orientation should be recorded. Preferably before surgery the patient is examined and the functional pelvic tilt is recorded and based on x-ray or other images taken the preferred inclination and version of the acetabular component to be implanted is planned. The functional pelvic tilt of the patient may differ from the tilt when the patient is positioned on an operating table. Therefore the functional tilt of patient's pelvis should be recorded prior to positioning the patient on the operating table and preferably in the position in which x-rays are taken. The recorded functional pelvic tilt should then be compared with the pelvic tilt when the patient is on the operating table and prepared to undergo surgery. In case a difference is noticed when comparing the original functional pelvic tilt with the actual pelvic tilt demonstrated when on the operating table and in the surgical position, the surgeon can decide to adapt the preplanned version and/or inclination angle of the acetabular cup implant.

In order to determine the original functional tilt of a patient's pelvis, first the three contact elements 7 should be positioned on the pelvis and with one of the ends contact the pelvis. Two of the contact elements 7 should be positioned on the anterior superior iliac spine, one on each side, of the pelvis 21 and the other contact element 7 should be positioned on the pubic bone of the pelvis 21. This can be done by sliding the guide elements 11 with respect to the spirit level carrier 5. Then the position of the slides 9 on the contact elements 7 should be adjusted and secured in position such that the bubble 3a of the spirit level 3 is in the center . The various positions of the moving elements 7 and 11 are then recorded. This procedure is undertaken prior to positioning the patient on the operating table and preferably when making images for acetabular cup component version and inclination planning, thereby taking into consideration patient's position as when on the operating table.

The aforementioned procedure is repeated after positioning the patient on the operating table and when prepared for surgery allowing at that moment to verify and compare the actual pelvic tilt and rotation with the original registration. In case a difference is noticed, the pelvic tilt and/or rotation can be adjusted or it may be decided to adapt the preplanned acetabular cup component version and/or inclination angle.

After removing the pelvic tilt verification device 1, the hip surgery can be executed in the routine manner.

Although the present invention is elucidated above on the basis of the given drawings, it should be noted that this invention is not limited whatsoever to the embodiments shown in the drawings. The invention also extends to all embodiments deviating from the embodiments shown in the drawings within the context defined by the claims.

## Claims

1. Pelvic tilt verification device (1) for determining a possible change of the original functional tilt of a patient's pelvis (21) and when positioned on an operating table to undergo hip surgery, comprising:
- a frame (5),
- guide elements (11) connected to the frame (5),
- a spirit level (3) connected to the frame (5),
- three pelvis contact elements (7),- three slides (9) which are slidably connected in a first direction to the frame (5), the slides (9) and the pelvic contact elements (7) are slidable relative to each other in a second direction different from the first direction,
- wherein the pelvic contact elements (7) are provided with markings in the direction in which the pelvic contact elements (7) are slidable for reading the position of the slides (9) on the pelvic contact elements (7), and
- wherein markings are present between the slides (9) and the frame (5) for determining the distance between the slides (9) and the frame (5),
**characterized in that**:
- one of the guide elements (11) is at an angle with respect to the other guide element(s) (11),
- the frame is a spirit level carrier (5),
- the spirit level is a bubble spirit level (3) which is mounted on the spirit level carrier (5), and
- the slides (9) are slidable along the pelvic contact elements (7).

2. Pelvic tilt verification device as claimed in claim 1, **characterized in that** the device comprises three guide elements (11) which are slidable in the spirit level carrier (5) and the slides (9) are attached to guide elements (11).

3. Pelvic tilt verification device as claimed in claim 1, **characterized in that** the slides (9) are slidable along guide elements (11) mounted on the spirit level carrier (5).

4. Pelvic tilt verification device as claimed in any of the preceding claims, **characterized in that** the first and second directions are at right angles to each other.

5. Pelvic tilt verification device as claimed in any of the preceding claims, **characterized in that** the pelvic tilt verification device (1) is provided with fixing means (13) for fixing the slides (9) and/or the guide elements (11) with respect to the pelvis contact elements (7) and/or the spirit level carrier (5) and the pelvis contact elements (7) and/or the guiding elements (11) are formed by elongated elements provided with markings.

6. Pelvic tilt verification device as claimed in any of the preceding claims, **characterized in that** the pelvis contact elements (7) and the guiding elements (11) are formed by straight bars, wherein two of the guiding elements (11) are parallel and the center line between these two parallel bars correspondents with the center of the spirit level, and the other guiding element (11) is perpendicular to said two parallel guiding elements (11) and is in line with the center of the spirit level.
